# EUROPEAN PATENT SPECIFICATION

(11) **EP 1 844 775 B1**
(45) Date of publication and mention of the grant of the patent: **11.08.2010**
(21) Application number: 06712757.1
(22) Date of filing: 01.02.2006
(51) Int. Cl.: A61K 31/422, A61K 31/4245, A61P 31/22, C07D 413/12

(54) **THERAPEUTIC AGENT FOR THE TREATMENT OF HERPES PROGENITALIS AFTER DEVELOPMENT OF LESIONS**
THERAPEUTISCHER WIRKSTOFF ZUR BEHANDLUNG VON HERPES PROGENITALIS NACH DER ENTWICKLUNG VON LÄSIONEN
AGENT THÉRAPEUTIQUE POUR LE TRAITEMENT DE L'HERPÈS GÉNITAL APRÈS LE DÉVELOPPEMENT DE LÉSIONS

(30) Priority: 02.02.2005 JP 2005027108
(43) Date of publication of application: 17.10.2007
(73) Proprietor: Astellas Pharma Inc., Tokyo 103-8411 (JP)
(72) Inventor: SUZUKI, Hiroshi, 2-chome, Chuo-ku Tokyo, 1038411 (JP); SUDO, Kenji, 2-chome, Chuo-ku Tokyo, 1038411 (JP)
(74) Representative: Bates, Philip Ian
(86) International application number: PCT/JP2006/301614
(87) International publication number: WO 2006/082820

(56) References cited:
- EP-A- 1 340 750
- WO-A-2005/014559
- WO-A1-02/38554
- WO-A1-03/095435
- WO-A1-2005/014559
- CRUTE J J ET AL: "Herpes simplex virus helicase-primase inhibitors are active in animal models of human disease" NATURE MEDICINE, NATURE PUBLISHING GROUP, NEW YORK, NY, US, vol. 8, no. 4, 1 April 2002 (2002-04-01), pages 386-391, XP002969973 ISSN: 1078-8956

## Description

### Technical Field

This invention relates to a novel medicament for genital herpes.

### Background Art

Genital herpes is a sexual infection which is developed by the infection with mainly herpes simplex virus type 2 (HSV-2) or partially herpes simplex virus type 1 (HSV-1), and it is considered that there are a large number of its patients mainly in Europe and America. Genital herpes develops a morbid state which is characterized by a severe pain after the infection with HSV, together with blister, erosion, ulcer formation and the like lesions accompanied by the multiplication of HSV in the periphery of the genitals, so that this is regarded as one of the diseases in which the patients feel physically and mentally great pain. At present, nucleic acid-based medicaments such as acyclovir (ACV) and its prodrugs valacyclovir (VCV), famciclovir (FCV) and the like are used as its therapeutic agents. However, it is generally known that when administration of these medicaments is started after the development of lesions, their effects are so weak that the therapeutic effects can rarely be expected. In addition, regarding the clinical dose of these nucleic acid-based anti-herpes medicaments, it is the present situation that a high dose of from several hundred mg to several g per day is administered. On the other hand, these nucleic acid-based agents are incorporated into the genomic DNA of the host by the DNA polymerase of the host so that apprehensions regarding their mutagenicity cannot be dispelled. Demand has been directed toward the development of a new medicament for genital herpes which considerably spoils quality-of-life of the patients, from which its effect can be expected even after the development of lesions.

The present inventors previously found an amide compound substituted with a thiazolylphenylcarbamoylmethyl group and with favorable anti-herpes virus activity, as represented by the following formula where the nitrogen atom of the amide group is substituted directly with an aromatic group aryl or heteroaryl group as ring A, or the salt thereof. Thus, the inventors filed a patent applications (Patent Reference 1 and Patent Reference 2). However, there is no disclosure about the activity on genital herpes after its onset. 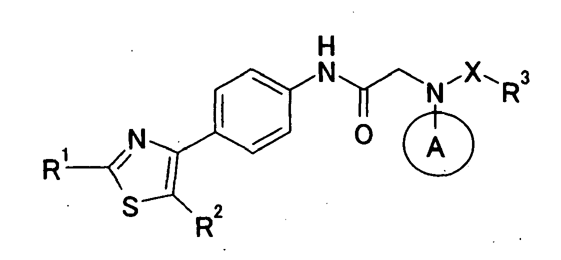 (In the formula, R¹ and R² represent -H, -lower alkyl,
-NRaRb or the like; A represents -aryl which may have a substituent(s), -heteroaryl which may have a substituent(s) or the like; X represents CO or SO₂; R³ represents -aryl which may have a substituent(s), -hetero ring which may have a substituent(s) or the like; see the Publication for details).

In addition, a compound represented by the following formula is disclosed in an application by the instant applicant and the like (Patent Reference 3) which was published after the priority date of the instant application. However, nothing is disclosed therein about the activity of said compound upon genital herpes after the onset. 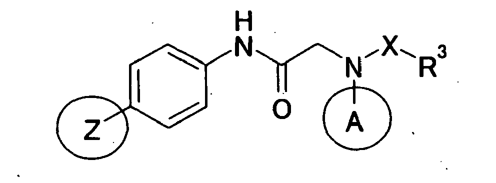 (In the formula, Z represents 1,2,4-oxadiazol-3-yl, 4-oxazolyl or the like, A represents an aryl group or the like which may have substituent(s), X represents CO or SO₂,
and R³ represents a hetero ring or the like which may have substituent(s). See said official gazette for details.)

Patent Reference 1: International Publication No. 02/38554
Patent Reference 2: International Publication No. 03/95435
Patent Reference 3: International Publication No. 05/014559 CRUTE J J ET AL : "Herpes simplex virus helicase-primase inhibitors are active in animal models of human disease, "NATURE MEDICINE, vol. 8, no.4, 1 April 2002 (2002-04-01), p.386-391

### Disclosure of the Invention

### Problems that the Invention is to Solve

To this day, great concern has been directed toward the creation of a medicament for genital herpes after the development of lesions, which is a non-nucleic acid-based, has high safety with low dose and is suited for oral administration.

### Means for Solving the Problems

The present inventors have conducted extensive studies on compounds having anti-herpes virus activity and, as a result, unexpectedly found that novel tetrahydro-2H-thiopyran-4-carboxamide derivatives which are characterized in that 1,2,4-oxadiazol-3-yl or 4-oxazolyl is introduced as the Z ring instead of the conventional amino-substituted thiazole ring, as shown in the following general formula (I), have a excellent anti-herpes virus activity. By further finding that these compounds show a excellent therapeutic effect on a model of genital herpes after the development of lesions, the invention has been accomplished.

That is, the invention relates to an agent for treating genital herpes after the onset, which comprises a novel N-{2-[(4-substituted phenyl)amino]-2-oxoethyl}tetrahydro-2H-thiopyran-4-carboxamide compound represented by the following general formula (I) as the active ingredient.

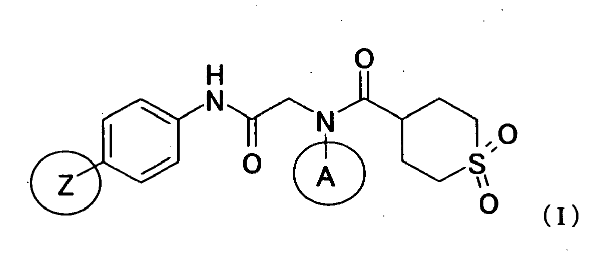

(Symbols in the formula have the following meanings
Z: 1,2,4-oxadiazol-3-yl or 4-oxazolyl group,
A: a phenyl group which is substituted by at least one methyl group and may further have 1 or 2 substituents selected from the group consisting of a methyl group and halogen atoms, or a 5-indanyl group. The same shall apply hereinafter.)

Particularly, the following compounds are desirable as the compounds represented by the general formula (I), which are the active ingredient of the medicament of the invention.
(1) A compound in which Z is a 1,2,4-oxadiazol-3-yl group.
(2) A compound in which Z is a 4-oxazolyl group.
(3) A compound in which A is a phenyl group which is substituted by at least one methyl group and may further have 1 or 2 substituents selected from the group consisting of a methyl group and halogen atoms.
(4) A compound in which A is a 5-indanyl group.
(5) A compound selected from the group consisting of
   N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,3-oxazol-4-yl)phenyl]amino}-2-oxoethyl)tetrahydro-2H-thiopyran-4-carboxamide 1,1-dioxide,
   N-(4-chloro-3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,5-difluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-fluoro-2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,2,4-oxadiazol-3-yl)phenyl]amino}-2-oxoethyl)tetrahydro-2H-thiopyran-4-carboxamide 1,1-dioxide,
   N-(3-fluoro-4-methylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-chloro-3-methylphenyl)-N-(2-{[4-(1,2,4-oxadiazol-3-yl)phenyl]amino}-2-oxoethyl)tetrahydro-2H-thiopyran-4-carboxamide 1,1-dioxide, and
   N-(3-fluoro-2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide.
   In addition, the invention also relates to a use of the N-{2-[(4-substituted phenyl)amino]-2-oxoethyl}tetrahydro-2H-thiopyran-4-carboxamide represented by the aforementioned general formula (I), for the manufacture of a medicament for treating genital herpes after development of lesions.

### Effect of the Invention

Since the compound (I) as the active ingredient of the present invention has excellent anti-herpes virus activity and exerts excellent therapeutic effect for genital herpes after development of lesions, the medicament of the present invention is useful for the treatment of genital herpes after development of lesions associated with infection.

### Best Mode for Carrying Out the Invention

The active ingredient of the invention, an N-{2-[(4-substituted phenyl)amino]-2-oxoethyl}tetrahydro-2H-thiopyran-4-carboxamide compound of the general formula (I), is further described.
In the invention, F, Cl, Br and I atoms may be exemplified as a "halogen atom".
The N-{2-[(4-substituted phenyl)amino]-2-oxoethyl}tetrahydro-2H-thiopyran-4-carboxamide compound of the invention represented by the general formula (I) also includes its hydrates, various solvates and polymorphic substances.

The following describes typical production methods of the compound (I) which is the active ingredient of the invention. In this connection, the production methods are not limited to the following examples.
In the following production methods, it is sometimes effective from the viewpoint of the production technique to replace a certain functional group depending on the type with an appropriate protective group, namely a group readily convertible to the functional group, at the stage of a raw material or intermediate. Afterwards, the protective group can be eliminated, if necessary, to obtain the desired compound. Examples of such a functional group includes an amino group, hydroxyl group, carboxyl group and the like. Protective groups thereof are, for example,
those described in Protective Groups in Organic Synthesis, the third edition (T. W. Green and P. G. M. Wuts, eds., JOHN WILLY & SONS, INC.). These may be appropriately used depending on the reaction conditions. For introducing and eliminating such protective groups, the methods described in the reference can be suitably applied.

### First Production Method

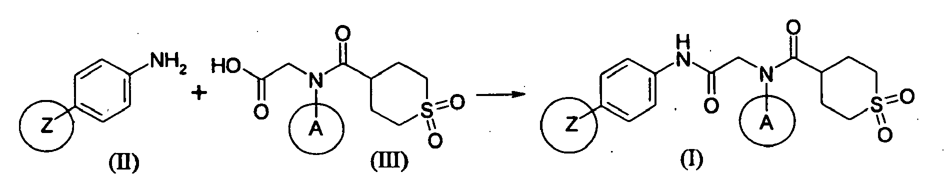

Compound (I) can be easily produced by subjecting Carboxylic Acid Compound (III) and Aniline Derivative (II) to an amidation reaction.

The amidation reaction can be carried out by general methods. For example, the method described in "Courses in Experimental Chemistry" edited by the Chemical Society of Japan, the fourth edition (Maruzen), Vol.22, pp.137-173 may be applicable. Preferably, the reaction is carried out by converting Carboxylic Acid Compound (III) to a reactive derivative such as an acid halide (acid chloride, etc.) or an acid anhydride, and then reacting the resulting reactive derivative with Aniline Derivative (II). In the case of using a reactive derivative of carboxylic acid, a base [an inorganic base such as potassium carbonate, sodium hydroxide, etc. or an organic base such as triethylamine (TEA), diisopropylethylamine, pyridine, etc.] is preferably added. In addition, the amidation reaction may be carried out by reacting carboxylic acid in the presence of a condensation agent [1-ethyl-3-(3-dimethylaminopropyl)carbodiimide (WSC), 1,1'-carbonylbis-1H-imidazole (CDI), etc.]. In this case, additives such as 1-hydroxybenzotriazole (HOBt), etc. may be added. The reaction temperature can be appropriately selected depending on the raw material compound used. The solvent usable includes those inert to the reaction, for example, aromatic hydrocarbon-series solvents such as benzene, toluene, etc.; ether-series solvents such as tetrahydrofuran (THF), 1,4-dioxane, etc.; halogenated hydrocarbon-series solvents such as dichloromethane, chloroform, etc.; amide-series solvents such as N,N-dimethylformamide (DMF), N,N-dimethylacetamide, etc.; basic solvents such as pyridine, etc.; and the like. The solvent is appropriately selected depending on the type of the raw material compound and the like, and can be used alone or as a mixture of two or more of them.

The aforementioned raw material compounds can be easily produced using known reactions, e.g., those described in "Courses in Experimental Chemistry" edited by the Chemical Society of Japan (Maruzen), in the pamphlet of the International Publication WO 02/38554, and the like. The typical production methods thereof are described below.

### Production method of Compound (III)

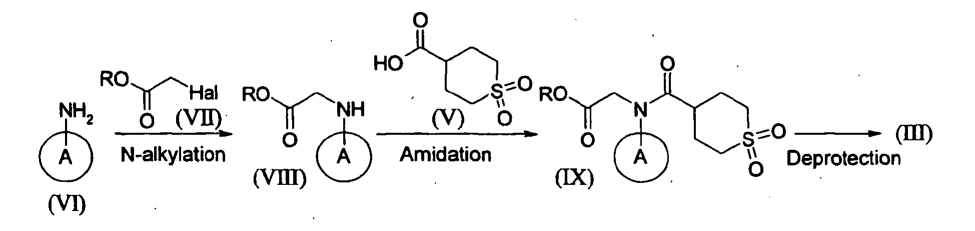

(In the formula, Hal means halogen, R means a group capable of forming an ester residue, such as a lower alkyl, an aralkyl, etc.)

In the reaction scheme above, amidation can be carried out in the same manner as in the first production method above.

N-alkylation of Compound (VI) can be carried out using Halogenated Alkyl Compound (VII) according to usual methods, e.g., the method described in the aforementioned "Courses in Experimental Chemistry", the fourth edition (Maruzen), Vol.20, pp.279-318. The reaction can be carried out under the temperature of from cooling to heating. Examples of the solvent usable include solvents inert to the reaction, for example, those exemplified for the amidation in the first production method, etc. The reaction is carried out preferably in the presence of a base such as potassium carbonate, sodium hydroxide, sodium hydride, etc. The amidation can be carried out in the same manner as in the first production method above. Herein, the amidation may be first carried out and subsequently, the N-alkylation may be carried out.

Deprotection for obtaining Carboxylic Acid Compound (III) can be carried out by appropriately applying a general method depending on the ester type. In the case of alkyl esters such as an ethyl ester, etc., the deprotection can be preferably carried out by treating them with a base such as sodium hydroxide aqueous solution, etc. In the case of aralkyl esters such as a benzyl ester, etc., the deprotection can be carried out by reducing them with palladium-carbon (Pd-C) under hydrogen atmosphere. The reactions can be carried out according to the method described in the aforementioned "Protective Groups in Organic Synthesis", the third edition.
A desired raw material compound can be produced by subjecting the compound with a certain substituent type to a substituent modification reaction well known to those skilled in the art.
The compound (I) of the present invention obtained in this manner is isolated and purified in its free form or as a salt thereof after a salt formation process by a general method. The isolation and purification are carried out by employing general chemical procedures such as extraction, concentration, evaporation, crystallization, filtration, recrystallization, various chromatographic techniques and the like.

The pharmaceutical composition of the present invention, which contains as effective components one type or two or more types of the compound (I) of the present invention, can be prepared according to a method usually used by using pharmaceutical carriers, excipients and the like for general use in this field. Administration thereof may be either oral via tablets, pills, capsules, granules, powders, liquids, etc. or parenteral dosing via injections such as intravenous injections, intramuscular injections, etc., external agents such as ointments, plasters, creams, jellies, cataplasm, sprays, lotions, eye drops, eye ointments, etc., suppositories, inhalation agents, and the like.

As the solid composition for oral administration, tablets, powders, granules and the like are used. In such a solid composition, one or more active substances are mixed with at least one inert excipient, for example, lactose, mannitol, glucose, hydroxypropyl cellulose, microcrystalline cellulose, starch, polyvinylpyrrolidone, magnesium metasilicate aluminate, etc. According to general methods, the composition may contain inert additives such as lubricants, e.g., magnesium stearate, etc.; disintegrators, e.g., sodium carboxymethyl starch, etc.; and dissolution auxiliary agents. The tablets or pills may be coated with sugar coating or stomach-soluble or enteric coating.

Examples of the liquid composition for oral administration include pharmaceutically acceptable emulsions, liquids, suspensions, syrups, elixirs, etc., in which inert solvents for general use such as purified water, ethanol, etc. can be incorporated. In addition to the inert solvents, the composition may further contain auxiliary agents such as solubilizing agents, moistening agents and suspending agents; sweetening agents; flavoring agents; aromatic agents and preservatives.

Examples of the injections for parenteral administration include sterile aqueous or non-aqueous liquids, suspensions and emulsions. The aqueous solvents include, for example, distilled water for injections and physiological saline. The non-aqueous solvents include, for example, propylene glycol, polyethylene glycol, vegetable oils such as olive oil, alcohols such as ethanol, Polysorbate 80 (name in the pharmacopeia) and the like. Such compositions may further contain isotonic agents, antiseptics, moistening agents, emulsifying agents, dispersing agents, stabilizers and dissolution auxiliary agents. These are sterilized by filtering through bacteria-retaining filters, by incorporating sterilizing agents, or by irradiation. Alternatively, these may be produced into a sterile solid composition and then dissolved or suspended in sterile water or sterile solvents for injections prior to use.

Examples of the external agents include ointments, plasters, creams, jellies, cataplasms, sprays, lotions, eye drops, eye ointments and the like. The external agent contains generally used ointment bases, lotion bases, aqueous or non-aqueous liquids, suspensions, emulsions and the like. As the ointment or lotion bases, polyethylene glycol, propylene glycol, white Vaseline, white beeswax, polyoxyethylene hardened castor oil, glycerin monostearate, stearyl alcohol, cetyl alcohol, lauromacrogol, sorbitan sesquioleate, carboxyvinyl polymer, and the like can be mentioned as examples.

Generally, the suitable daily dose of the compound (I) of the present invention, which is the active ingredient of the present invention, is about 0.001 to 50 mg/kg/body weight, preferably 0.01 to 30 mg/kg/body weight, more preferably 0.05 to 10 mg/kg/body weight, for oral administration. For intravenous administration, the daily dose is about 0.0001 to 10 mg/kg/body weight, preferably 0.001 to 1.0 mg/kg/body weight. The dose is administered once or in separate portions per day, and is appropriately determined depending on each case, in terms of the symptom, age, sex and the like. When the compound (I) is to be used as an external agent, the agent containing the compound of the invention in an amount of 0.0001 to 20%, preferably 0.01 to 10%, is desirable. The external agent is administered locally once or in separate portions per day depending on the symptom.

### Examples

Effects of the medicament of the invention were confirmed by the following pharmacological tests.

### Example 1 Therapeutic activity upon guinea pig HSV-2 genital herpes model after the development of lesions

*In vivo* activity of the pharmaceutical composition of the present invention was tested using an HSV-2 infected guinea pig vagina genital herpes model in reference to the method of L. Stanberry et al. (Journal of Infectious Disease, 1982, 146, 397 - 404). An absorbent cotton swab was impregnated with an HSV-2 virus suspension (HSV-2 strain G 1.25 x 10⁵ PFU/ml) and inserted into the vagina of a Hartley female guinea pig (4 weeks of age) under diethyl ether anesthesia, thereby infecting it with HSV-2. Vagina lesions and general conditions caused by the HSV-2 infection were scored based on the following 6 steps and evaluated every day from the infected day (0 day) to 9 days after the infection.
Score 0: no lesions
Score 1: formation of approximately 1 or 2 small blisters which can be observed with the naked eye
Score 2: formation of blisters in 10 to 50% per vaginal visible area
Score 3: formation of blisters in 50 to 100% per vaginal visible area
Score 4: formation of ulcers in 10 to 50% per vaginal visible area
Score 5: formation of ulcers in 50 to 100% per vaginal visible area
Score 6: paralysis of hind legs or death

Only guinea pigs in which the vaginal lesion score was judged to be 1 or 2 and development of lesions therefore was found were selected after the development of vaginal lesions by HSV-2 infection, namely 4 days after the infection, their grouping was carried out in such a manner that average values of the scores became even, and then administration of each compound to be tested was started. Each compound to be tested was made into a methyl cellulose suspension and orally administered twice a day for 5 days of from the 4th day to 8th day after the infection. Additionally, in order to compare degrees of the therapeutic effect due to difference in the administration starting period, a comparative test was simultaneously carried out in which the administration was started before the development of vaginal lesions (3 hours after the HSV-2 infection) and the administration was carried out in the same manner until 4 days after the infection. Each test was carried out using 10 animals per group of the guinea pigs and using VCV as the comparative compound.

Regarding the average vaginal lesion score of 9 days after the HSV-2 infection, the dose of each compound to be tested which inhibits 50% of the score of the placebo administration group (ED₅₀ value; mg/kg, twice a day) was calculated, with the results shown in the following table.

**[Table 1]**

| | Compound to be tested | Lesion inhibitory activity (ED₅₀; mg/kg) (4 to 8 days of administration after infection) | Comparative test (administration before the development of lesions) (ED₅₀; mg/kg) (0 to 4 days of administration after infection) |
|---|---|---|---|
| (1) | Compound of Preparation 2 | 3.1 | 1.3 |
| | Comparative compound: VCV | >300 | 117 |
| (2) | Compound of Preparation 27 | 2.5 | 1.2 |
| | Comparative compound: VCV | >300 | 50 |

The VCV as the comparative compound dose-dependently inhibited the genital herpes lesions in the comparative test in which its administration was carried out before the development of genital herpes lesions (5 days of administration from 0 to 4 days after the HSV-2 infection), and its ED₅₀ values were 117 and 50 (mg/kg, twice a day). However, when administration of VCV was started after the development of genital herpes lesions, its ED₅₀ value was calculated to be 300 (mg/kg, twice a day) or more in each test, so that the lesion improving effect of VCV was considerably attenuated. This result reflected the clinical problem in that when an already existing anti-herpes drug is administered after the development of genital herpes lesions, its effect is so weak that its sufficient therapeutic effect cannot be obtained.

On the other hand, ED₅₀ values of the compounds of Preparations 2 and 27 of the invention were excellent values of 3.1 and 2.5 (mg/kg, twice a day) which were almost identical to the values 1.3 and 1.2 (mg/kg, twice a day) by their administration before the development of lesions in the comparative test examples. These results show that, different from the already existing anti-herpes virus agents, the medicament of the present invention exerts excellent improving effect even by its administration after the development of genital herpes lesions.

As described in the above, it was confirmed that, different from the already existing anti-herpes virus agents, the compounds of the present invention are possessed of excellent therapeutic activity upon genital herpes after the development of lesions.
Since the compounds of the invention are not nucleic acid-based and show an excellent activity, it was expected that they can become high safety medicaments for genital herpes after the development of lesions.

### (Preparations)

Production examples of the compound (I), which is an active ingredient of the present invention, are shown below as Preparations. Herein, many of the raw material compounds for use in the following reactions are known in the pamphlet of the Patent Reference 1 (International Publication WO 02/38554) and the like, and can therefore be readily available according to the methods described in these known references. Production examples of novel compounds among the raw materials are shown below in Reference Examples.
Reference Example 1:
   5% Palladium-carbon powder was added to an ethanol-tetrahydrofuran mixed suspension of 4-(4-nitrophenyl)-1,3-oxazol and stirred for 12 hours at room temperature in a hydrogen atmosphere. The reaction solution was filtered through Celite and the filtrate was evaporated under reduced pressure. The resulting crude product is purified with a silica gel column chromatography to obtain [4-(1,3-oxazol-4-yl)phenyl]amine (pale yellow solid). Electron Impact-MS(M)⁺: 160.
Reference Example 2:
   Potassium carboxylate and ethyl bromoacetate were added to a DMF solution of 4-methylaniline and heated while stirring. The reaction mixture was added with water and ethyl acetate. After the organic layer was separated, washed and dried, the solvent was evaporated under reduced pressure to obtain a crude product. The crude product was dissolved in methylene chloride, and pyridine, tetrahydro-2H-thiopyrane-4-carbonyl chloride 1,1-dioxide were added to the resulting solution and stirred. After the reaction solution was concentrated, 1M hydrochloric acid and chloroform were added. The organic layer separated was washed and dried and the solvent was evaporated under reduced pressure. The resulting crude product was purified with a silica gel column chromatography to obtain ethyl {[(1,1-dioxotetrahydro-2H-thiopyran-4-yl)carbonyl](4-methylphenyl)amino}acetate (colorless oily product). FAB-MS [(M+H)⁺]: 354.
Reference Examples 3 to 15:
   Compounds of Reference Examples 3 to 15, which are described in Table 2 below, were obtained in the same manner as in Reference Example 2.

Preparation 1:
To an ethanol (10 ml) solution of ethyl {(2,6-dimethylphenyl)[(1,1-dioxide tetrahydro-2H-thiopyran-4-yl)carbonyl]amino}acetate (735 mg) was added aqueous 1M sodium hydroxide solution (2.3 mL). The mixture was stirred at room temperature for 5 hours. After 1M hydrochloric acid was added to the reaction mixture to make the solution acidic, water and chloroform were added thereto to separate the organic layer. Further, the organic layer was dried over anhydrous sodium sulfate and filtered, and then, the solvent was evaporated under reduced pressure. After the resulting crude carboxylic acid product was dissolved in chloroform (15 ml), WSC · HCl (422 mg) and [4-(1,3-oxazol-4-yl)phenyl]amine (320 mg) were added sequentially to the resulting solution, which was stirred at room temperature for 4 hours. After a saturated sodium hydrogencarbonate aqueous solution and chloroform were added to the reaction solution, the organic layer was separated. The organic layer was washed with a saturated sodium chloride solution, dried over anhydrous magnesium sulfate and filtered, from which the solvent was evaporated under reduced pressure. The resulting crude product was rinsed in hexane-ethyl acetate (= 3/2), and then recrystallized from ethanol, to obtain N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide (colorless crystal) in a yield of 610 mg.
Preparations 2 - 40:
   Compounds of Preparations 2 to 40 shown in Tables 3 to 10 below were obtained in the same manner as in Preparation 1.

The physicochemical properties of the compounds of Reference Examples are shown in Table 2, while Tables 3 to 10 show the structures and physicochemical properties of the compounds of Preparations.

Abbreviations in the tables indicate as follows.
Ref: Reference Example; Ex: Preparation; Dat: physicochemical properties {F+: FAB-MS [(M+H)⁺]; F-: FAB-MS [(M-H)⁻]; N1: ¹H-NMR (DMSO-d₆, TMS internal standard); mp: melting point (°C), solvent for crystallization is shown in the parentheses}; Ph: phenyl; Me: methyl; Et: ethyl; and iPr: isopropyl. Herein, the numerical figure before each substituent indicates the position for its substitution. For example, 3,4-(Cl)₂-5-F-Ph indicates a 3,4-dichloro-5-fluorophenyl group.

**[Table 2]**

| | | | | | | | |
|---|---|---|---|---|---|---|---|
| 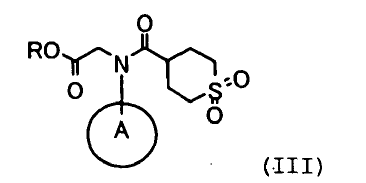 | | | | | | | |

| Ref | A | R | Dat | Ref | A | R | Dat |
|---|---|---|---|---|---|---|---|
| 3 | 2,3-(Me)₂-Ph | Et | F+: 368 | 4 | 4-Me-Ph | Et | F+: 354 |
| 5 | 2,5-(Me)₂-Ph | Et | F+: 368 | 6 | 3-Me-Ph | Et | F+: 354 |
| 7 | 3,4-(Me)₂-Ph | Et | F+: 368 | 8 | 2-Me-Ph | Et | F+: 354 |
| 9 | 2,4,6-(Me)₃-Ph | Et | F+: 382 | 10 | 2,4-(Me)₂-Ph | Et | F+: 368 |
| 11 | 4-F3-Me-Ph | Et | F+: 372 | 12 | 2,6-(Me)₂-Ph | Et | F+: 368 |
| 13 | 3-Br-4-Me-Ph | Et | F+: 432, 434 | 14 | 4-F-2,6-(Me)₂-Ph | Et | F+: 386 |
| 15 | 3,5-(Me)₂-Ph | Et | F+: 368 | | | | |

**[Table 3]**

| | | |
|---|---|---|
| 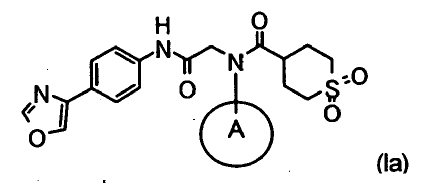 | | |

| **Ex** | **A** | **Dat** |
|---|---|---|
| 1 | 2,6-(Me)₂-Ph | F+: 482 N1: 1.87 2.42(5H, m), 2.13(6HX0.1, s), 2.33(6HX0.9, s), 2.97-3.27(4 H, m), 4.19(2HX0.9, s), 4.48(2HX0.1, s), 7.07-725(3H, m), 7.62-7 .66(2H, m), 7.72-7.75(2H, m), 8.43(1H, d), 8.54(1H, d), 10.15(1H, brs) mp: 224-227 (EtOH) |
| 2 | 4-Me-Ph | F+:468 N1: 1.98-2.06(4H, m), 2.34(3H, s), 2.68-2.70(1H, m), 2.97-3.02(4H, m), 4.35(2H, s), 7.28 (2H, d), 7.36(2H, d), 7.63-7.66(2H, m), 7.7 2-7.76(2H, m), 8.43(1H, s), 8.54(1H, s), 10.14(1H, s) mp: 199-201 (EtOH-H₂O) |
| 3 | 3-Me-Ph | F+: 468 N1: 2.01-2.09(4H, m), 2.35(3H, s), 2.71 (1H, m), 2.933.06(4H, m), 4 .36(2H, s), 7.17-7.38 (4H, m), 7.64(2H, d), 7.73(2H, d), 8.43(1H, d), 8.54(1H, d), 10.15(1H, s) |
| 4 | 2-Me-Ph | F+: 468 N1: 1.88-2.15(4H, m), 2.15(3HX0.1, s), 2.26(3HX0.9, s), 2.41-2.46(1 H, m), 2.83-3.05(4H, m), 3.86(1HX0.9, d), 4.20(1HX0.1, d), 4.74( 1HX0.9, d), 4.84(1HX0.1, d), 7.09-7.77(8H, m), 8.43(1H, d), 8.53( 1H, d), 10.14(1HX0.9, s), 10,19(1HX0,1, s) mp: 220-221 (EtOH) |

**[Table 4]**

| | | |
|---|---|---|
| 5 | 2,3-(Me)₂-Ph | F+: 482 N1: 1.85-2.12(4H, m), 2.03(3HX0.1, s), 2.15(3HX0.9, s), 2.25(3HX0. 1, s), 2.31(3HX0.9, s), 2.42-2.47(1H, m), 2.83-2.90(1H, m), 3.00-3 22(3H, m), 3.84(1HX0.9, d), 4.16(1HX0.1, d), 4.72(1HX0.9, d), 4. 84(1HX0.1, d), 7.07-7.36(3H, m), 7.62-7.66(2H, m), 7.71-7.76(2H, m), 8.43(1H, brs), 8.54(1H, d), 10.12(1HX0.9, s), 10.16(1HX0.1, s) mp: 185-187 (EtOH) |
| 6 | 2,4(Me)₂-Ph | F+: 482 N1:1.88-2.50(5H, m), 2.09(3HX0.1, s), 221(3HX0.9, s), 2.25(3HX0. 1, s), 2.30(3HX0.9, s), 2.85-320(4H, m), 3.81(1HX0.9, d), 4.17(1 HX0.1, d), 4.72(1HX0.9, d), 4.81(1HX0.1, d), 6.97-7.39(3H, m), 7. 62-7.66(2H, m), 7.72-7.76(2H, m), 8.43(1H, s), 8.54(1H, s), 10.11 (1HX0.9, s), 10.17(1HX0.1, s) mp: 176-177 (EtOH) |
| 7 | 2,5-(Me)₂-Ph | F+: 482 N1: 1.86-2.51(5H, m), 2.08(3HX0.1, s), 2.20(3HX0.9, s), 2.22(3HX0. 1, s), 2.30(3HX0.9, s), 2.87-3.26(4H, m), 3.84(1HX0.9, d), 4.21(1 1HX0.1, d), 4.70(1HX0.9, d), 4.80(1HX0.1, d), 6.92-7.32(3H, m), 7. 63-7.65(2H, m), 7.72-7.76(2H, m), 8.43(1H, s), 8.54(1H, s), 10.12 (1HX0.9, s), 10.17(1HX0.1, s) mp: 201-202 (EtOH) |
| 8 | 3,4(Me)₂-Ph | F+: 482 N1: 1.92-2.08(4H, m), 2.09(3H, s), 2.24(3H, s), 2.71(1H, s), 2.94-3. 06(4H, m), 4.33(2H, S), 7.17-7.24(3H, m), 7.64(2H, d), 7.73(2H, d), 8.43(1H, s), 8.54(1H, s), 10.12(1H, s) |
| 9 | 3,5-(Me)₂-Ph N1: | F+: 482 1.96-2.14(4H, m), 2.30(6H, s), 2.73(1H, m), 2.95-3.04(4H, m), 4 .33(2H, S), 7.02(1H, s), 7.08(2H, s), 7.64(2H, d), 7.73(2H, d), 8.4 3(1H, s), 8.54(1H, s), 10.12(1H, s) mp: 205-206 (EtOH) |

**[Table 5]**

| | | |
|---|---|---|
| 10 | 2,4,6-(Me)₃-Ph | F+: 496 N1: 1.87-2.45(5H, m), 2.08(3HX0.1, s), 2.09(6HX0.1, s), 227(3HX0. 9, s), 2.28(6HX0.9, s), 3.01-3.26(4H, m), 4.16(2HX0.9, s), 4.44(2 HX0.1, s), 6.88(2HX0.1, s), 7.01 (2HX0.9, s), 7.61-7.65(2H, m), 7. 71-7.75(2H, m), 8.43(1H, s), 8.54(1H, s), 10.12(1HX0.9, s), 10.14 (1HX0.1, s) mp: 237-238 (EtOH) |
| 11 | 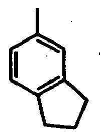 | F+: 494 N1: 2.01-2.08(6H, m), 2.70-3.06(9H, m), 4.34(2H, s), 7.13-7.32(3H, m), 7.64(2H, d), 7.73(2H, d), 8.43(1H, s), 8.54(1H, s), 10.13(1H, s) |
| 12 | 3-Cl-4-Me-Ph | F+: 502 N1: 2.01-2.06(4H, m), 2.36(3H, s), 2.68-2.75(1H, m), 3.01-3.06(4H, m), 4.37(2H, S), 7.37-7.40(1H, m), 7.46(1H, d), 7.60-7.66(3H, m), 7.74(2H, d), 8.44(1H, s), 8.55(1H, s), 10.18(1H, s) mp: 146-148 (EtOH) |
| 13 | 4-Cl-3-Me-Ph | F+: 502 N1: 2.00-2.06(4H, m), 2.36(3H, s), 2.68-2.75(1H, m), 3.01-3.04(4H, m), 4.36(2H, S), 7.33-7.36(1H, m), 7.48-7.52(2H, m), 7.64(2H, d), 7.73(2H, d), 8.43(1H, s), 8.54(1H, s), 10.18(1H, s) mp: 133-135 (EtOH) |
| 14 | 3-F-4-Me-Ph | F+: 486 N1: 2.00-2.05(4H, m), 2.26(3H, s), 2.70-2.77(1H, m), 3.01-3.03(4H, m), 4.36(2H, S), 724-7.26(1H, m), 7.327.41(2H, m), 7.64(2H, d), 7.73(2H, d), 8.43(1H, s), 8.54(1H, s), 10.17(1H, s) mp: 135-137 (EtOH) |
| 15 | 3-Br-4-Me-Ph | F+: 546, 548 N1: 2.00-2.06(4H, m), 2.38(3H, s), 2.68-2.74(1H, m), 3.01-3.04(4H, m), 4.36(2H, S), 7.41-7.47(2H, m), 7.64(2H, d), 7.73-7.76(3H, d), 8.43(1H, s), 8.54(1H, s), 10.18(1H, s) mp: 154-155 (EtOH) |

**[Table 6]**

| | | |
|---|---|---|
| 16 | 5-F-2-Me-Ph | F+: 486 N1: 1.88-2.15(4H, m), 2.11(3HX0.1, s), 2.23(3HX0.9, s), 2.45-2.49(1 H, m), 2.96-3.16(4H, m), 3.92(1HX0.9, d), 4.27(1HX0.1, d), 4.70( 1HX0.9, d), 4.82(1HX0.1, d), 6.95-6.98(1HX0.1, m), 7.06-7.10(1H X0.1, m), 7.20-7.25(1HX0.9, m), 7.29-7.33(1HX0.1 m), 7.37-7.7.4 0(1HX0.9, m), 7.42-7.46(1HX0.9, m), 7.65(2H, d), 7.74(2H, d), 8. 43(1H, s), 8.54(1H, s), 10.18(1HX0.9, s), 10.23(1HX0.1, s) mp: 235-236 (EtOH) |
| 17 | 3-F-2,4-(Me)₂-Ph | F+: 500 N1: 1.88-2.23(4H, m), 2.03(3HX0.1, s), 2.16(3HX0.9, s), 220(3HX0. 1, s), 2.26(3HX0.9, s), 2.47 2.54(1H, m), 2.87-3.17(4H, m), 3.91 (1 HX0.9, d), 4.25(1HX0.1, d), 4.66(1HX0.9, d), 4.80(1HX0.1, d), 6.8 8 (1HX0.1, d), 7.10(1HX0.1, dd), 7.21 (1HX0.9, dd), 7.28(1HX0.9, d), 7.64(2H, d), 7.73(2H, s), 8.43(1H, s), 8.54(1H, s), 10.14(1HX 0.9, s), 10.20(1HX0,1, s) mp: 185-186 (EtOH) |
| 18 | 4-F-3,5-(Me)₂-Ph | F+: 500 N1: 2.00-2.05(4H, m), 2.24(6H, s), 2.67-2.74(1H, m), 3.00-3.04(4H, m), 4.33(2H, S), 7.23(2H, d), 7.65 (2H, d), 7.74(2H, d), 8.43(1H, s), 8.54(1H, s), 10.15(1H. s) mp: 188-189 (EtOH) |
| 19 | 3,5-F₂-4-Me-Ph | F+: 504 N1: 1.99-2.05(4H, m), 2.17(3H, s), 2.75-2.82(1H, m), 2.99-3.10(4H, m), 4.37(2H, S), 7.28(2H, d), 7.65 (2H, d), 7.74(2H, d), 8.44(1H, s), 8.55(1H, s), 10.21(1H, s) mp: 221-223 (EtOH) |
| 20 | 2-F-4-Me-Ph | F+: 486 N1: 1:89-2.11(4H, m), 2.30(3HX0.1, s), 2.36(3HX0.9, s), 2.60-2.68(1 H, m), 3.01-3.26(4H, m), 3.94(1HX0.9, d), 4.02(1HX0.1, d), 4.50( 1HX0.1, d), 4.76(1HX0.9, d), 7.00(1HX0.1, d), 7.09(1HX0.1, d), 7. 12(1HX0.9, d), 724(1HX0.9, d), 7.38 (1HX0.1, dd), 7.50(1HX0.9, dd), 7.63(2H, d), 7.73(2H, d), 8.44(1H, s), 8.55(1H, s), 10.17(1HX 0.9, s), 10.23(1HX0.1, s) |

**[Table 7]**

| | | |
|---|---|---|
| 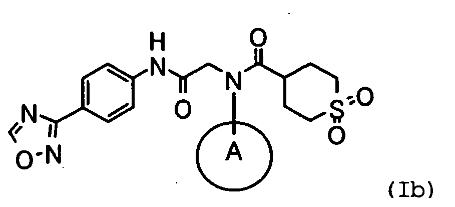 | | |

| **Ex** | **A** | **Dat** |
|---|---|---|
| 21 | 4-Me-Ph | F+: 469 N1: 1.94-2.11(4H, m), 2.34(3H, s), 2.65-2.75(1H, m), 2.92-3.08(4H. m ), 4.38(2H, s), 7.28(2H. d), 7.37(2H, d), 7.79(2H, d), 8.00(2H, d), 9. 66(1H, s), 10.38(1H, s) mp: 203-205 (EtOH) |
| 22 | 3-Me-Ph | F-: 467 N1: 1.96-2.11(4H, m), 2.35(3H, s), 2.65-2.76(1H, m), 2.92-3.09(4H, m ), 4.39(2H, s), 7.20-7:39(4H, m), 7.79(2H, d), 8.00(2H, d), 9.66(1H, s), 10.38(1H, s) |
| 23 | 2-Me-Ph | F+: 469 N1: 1.88-2.26(4H+3H, m), 2.42-2.52(1H, m), 2.84-3.18(4H, m), 3.91(1 H × 0.9, d), 4.44(1H × 0.1, d), 4.75(1H × 0.9, d), 4.87(1H × 0.1, d), 7. 08-7.54(4H, m), 7.75-7.81(2H, m), 7.97-8.04(2H, m), 9.66(1H × 0.9, s), 9.67(1H × 0.1, s). 10.37(1H × 0.9, s), 10.41 (1 H × 0.1, s) mp: 216-217 (MeOH) |
| 24 | 2,3-(Me)₂-Ph | F-: 481 N1: 1.83-2.31 (4H+3H+3H, m), 2.42 2.54(1H, m), 2.82-3.16(4H, m), 3. 88(1H × 0.9, d), 4.19(1H×0.1, d), 4.72(1H×0.9, d), 4.87(1H × 0.1, d), 7.05-7.37(3H, m), 7.75-7.80(2H, m), 7.97-8.03(2H, m), 9.66(1H × 0.9, s), 9.66(1H×0.1, s), 10.35(1H×0.9, s), 10.38(1H×0.1, s) mp: 223-225 (EtOH) |
| 25 | 2,4-(Me)₂-Ph | F-: 481 N1: 1.84-2.33(4H+3H+3H, m), 2.422.52(1H, m), 2.843.1.9(4H, m), 3. 86(1H×0.9, d), 4.21(1H×0.1, d), 4.73(1H×0.9, d), 4,84(1H×0.1, d), 6.95-7.40(3H, m), 7.75-7.81(2H, m), 7.98-8.02(2H, m), 9.66(1H × 0.9, s), 9.66(1H×0.1, s), 10.35(1H×0.9, s), 10.39(1H×0.1, s) mp: 139-141 (EtOH) |

**[Table 8]**

| | | |
|---|---|---|
| 26 | 2,5-(Me)₂-Ph | F-: 481 N1: 1.84-2.32(4H+3H+3H, m), 2.42-2.52(1H, m), 2.87-3.18(4H, m), 3. 89(1H×0.9, d), 4.25(1H × 0.1, d), 4.72(1H × 0.9, d), 4.83(1H × 0.1, d), 6.92-7.34(3H, m), 7.76-7.82(2H, m), 7.98-8.04(2H, m), 9.66(1H × 0.9, s), 9.67(1H × 0.1, s), 10.37(1H × 0.9, s), 10.39(1H × 0.1, s) mp: 214-217 (EtOH) |
| 27 | 2,6-(Me)₂-Ph | F-: 481 N1: 1.88-2.42(5H+6H, m), 2.98-3.27(4H, m), 422(2H × 0.86, s) 4.51(2 H × 0.14, s), 7.17.3(3H, m), 7.76-7.81(2H, m), 7.99-8.03(2H, m), 9. 66(1H, s), 10.38(1H, s) mp:220-222(EtOH-H₂O) |
| 28 | 3,4-(Me)₂-Ph | F+: 483 N1: 1.97 2.20(4H, m), 2.24(6H, s), 2.67-2.76(1H, m), 2.96-3.30(4H. m ), 4.37(2H, s), 7.17 7.27(3H, m), 7.79(2H, d), 8.00(2H, d), 9.66(1H, s), 10.36(1H, s) mp: 141-143 (EtOH) |
| 29 | 3,5-(Me)₂-Ph | F+: 483 N1: 1.98-2.12(4H, m), 2.30(6H, s), 2.65-2.78(1H, m), 2.93-3.10(4H, m ), 4.36(2H, s), 7.00-7.12(3H, m), 7.79(2H, d), 8.00(2H, d), 9.66(1H, s), 10.37(1H, s) mp: 197-198 (iPrOH) |
| 30 | 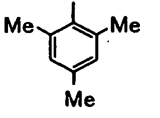 | F-: 495 N1: 1.83-2.52(4H+9H+1H, m), 2.99-3.26(4H, m), 4.18(2H × 0.9, s), 4. 48(2H × 0.1, s), 6.88(2H × 0.1, s), 7.01(2H × 0.9, s), 7.74-7.82(2H, m), 7.94-8.03(2H, m), 9.66(1H, s), 10.36(1H, s) mp: 188-190 (EtOH) |
| 31 | 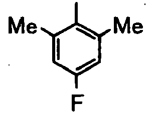 | F-: 499 N1: 1.82-2.44(6H+5H, m), 2.98-3.30(4H, m), 421(2H × 0.85, s), 4.50( 2H × 0.15, s), 6.95(2H × 0.15, d), 7.08(2H × 0.85, d), 7.75-7.82(2H, m), 7.97-8.04(2H, m), 9.66(1H × 0.85, s), 9.66(1H × 0.15, s), 10.40( 1H, brs) mp: 226-229 (EtOH-MeCN-H₂O) |

**[Table 9]**

| | | |
|---|---|---|
| 32 | 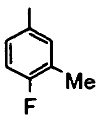 | F+: 487 N1: 1.97-2.11(4H, m), 2.26(3H, brs), 2.63-2.74(1H, m), 2.95-3.07(4 H, m), 4.38(2H, s), 7.21-7.45(3H, m), 7.79(2H, d), 8.00(2H, d), 9. 66(1H, s), 10.39(1H, s) mp: 136-138 (EtOH) |
| 33 | 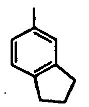 | F-:493 N1: 1.96-2.20(6H, m), 2.70-2.78(1H, m), 2.84-3.08(8H, m), 4.37(2H, s), 7.04-7.33(3H, m), 7.79(2H, d), 8.00(2H, d), 9.66(1H, s), 10.37 (1H, s) |
| 34 | 4-Me-3-Br-Ph | F-: 546 N1: 1.96-2.16(4H, m), 2.38(3H, s), 2.66-2.77(1H, m), 2.96-3.08(4H, m), 4.39(2H, s), 7.40-7.49(2H, m), 7.73-7.82(3H, m), 8.00(2H, d), 9.66(1H, s), 10.41(1H, s) mp: 203-206 (iPrOH) |
| 35 | 3-F-4-Me-Ph | F+: 487 N1: 1.97-2.07 (4H, m), 226 (3H, s), 2.69-2.77 (1H, m), 2.99-3.03 (4H, m), 4.39 (2H, s), 7.22-7.28 (1H, m), 7.31-7.42 (2H, m), 7.80 (2H, d), 7.99 (2H, d), 9.66 (1H, s), 10.40 (1H, s) mp: 200-203 (EtOH) |
| 36 | 3-Cl-4-Me-Ph | F+: 503 N1: 1.97-2.11 (4H, m), 2.36 (3H, s), 2.65-2.78 (1H, m), 2.97-3.08 ( 4H, m), 4.39 (2H, s); 7.39 (1H, dd), 7:45 (1H, d), 7.60 (1H, d), 7. 80 (2H, d), 7.99 (2H, d), 9.65 (1H, s), 10.40 (1H, s) mp: 204-205 (EtOH) |
| 37 | 4-Cl-3-Me-Ph | F+: 503 N1: 1.95-2.09 (4H, m), 2.36 (3H, s), 2.65-2.76 (1H, m), 2.95-3.07 ( 4H, m), 4.39 (2H, s), 7.36 (1H, dd), 7.48 (1H, d), 7.51. (1H, d), 7. 80 (2H, d), 7.99 (2H, d), 9.66 (1H, s), 10.40 (1H, s) mp: 196-199 (EtOH) |
| 38 | 4-F-3,5-(Me)₂-Ph | F+: 501 N1: 1.94-2.12(4H, m), 2.24(6H, s), 2.64-2.74(1H, m), 2.94-3.08(4H, m), 4.35(2H, s), 7.23(2H, d), 7.79(2H, d), 7.99(2H, d), 9.66(1H, s) , 10.38(1H, s) |

**[Table 10]**

| | | |
|---|---|---|
| 39 | 3-F-2,4-(Me)₂-Ph | F+: 501 N1: 1.84-2.34(4H+3H+3H, m), 2.48-2.55(1H, m), 2.85-3.22(4H, m), 3.98(1H × 0.9, d), 4.30(1H × 0.1, d), 4.65(1H × 0.9, d), 4.81 (1H × 0 1, d), 7.22(1H, t), 7.27(1H, d), 7.78(2H, d), 7.98(2H, d), 9.66(1H, s), 10.37(1H × 0.9, s), 10.51(1H × 0.1, s) |
| 40 | F+: 487 2-F-4-Me-Ph | N1: 1.90-2.18(4H, m), 2.30(3HX0.1, s), 2.36(3HX0.9, s), 2.62-2.68(1 H, m), 3.01-3.23(4H, m), 3.99(1H, d), 4.77(1H, d), 7.13(1H, d), 7. 25(1H, d), 7.50(1H, dd), 7.77(2H, d), 7.99(2H, d), 9.66(1H, s), 10. 40(1HX0.9, s), 10.45(1HX0.1, s) mp: 197-198 (EtOH) |

### Industrial Applicability

Since the compound (I), which is the active ingredient of the present invention, has excellent anti-herpes virus activity and exerts excellent therapeutic effect upon genital herpes after the development of lesions, the medicament of the present invention is useful for the treatment of genital herpes after the development of lesions accompanied by its infection.

## Claims

1. A medicament for use in treating genital herpes after the development of lesions, which comprises an N-{2-[(4-substituted phenyl)amino]-2-oxoethyl}tetrahydro-2H-thiopyran-4-carboxamide compound represented by the following general formula (I) as the active ingredient 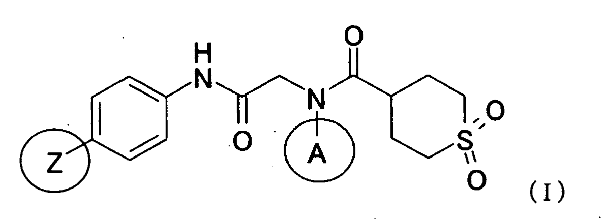 (symbols in the formula have the following meanings Z: a 1,2,4-oxadiazol-3-yl or 4-oxazolyl group,
A: a phenyl group which is substituted by at least one methyl group and may further have 1 or 2 substituents selected from the group consisting of a methyl group and halogen atoms, or a 5-indanyl group).

2. The medicament for uses described in claim 1, wherein Z is a 1,2,4-oxadiazol-3-yl group.

3. The medicament for uses described in claim 1, wherein Z is a 4-oxazolyl group.

4. The medicament for uses described in claim 1, wherein A is a phenyl group which is substituted by at least one methyl group and may further have 1 or 2 substituents selected from the group consisting of a methyl group and halogen atoms.

5. Use of the N-{2-[(4-substituted phenyl)amino]-2-oxoethyl}tetrahydro-2H-thiopyran-4-carboxamide represented by the formula (I) described in claim 1, for the manufacture of a medicament for treating genital herpes after the development of lesions.

## Patentansprüche

1. Medikament zur Verwendung bei der Behandlung von Genitalherpes nach der Entwicklung von Läsionen, das als Wirkstoff eine durch die folgende allgemeine Formel (I) repräsentierte N-{2-[(4-substituiertes Phenyl)amino]-2-oxoethyl}tetrahydro-2H-thiopyran-4-carboxamid-Verbindung umfasst: 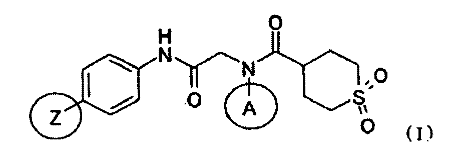 (Symbole in der Formel haben die folgende Bedeutung:
Z: eine 1,2,4-Oxadiazol-3-yl- oder 4-Oxazolylgruppe,
A: eine Phenylgruppe, die durch wenigstens eine Methylgruppe substituiert ist und ferner 1 oder 2 Substituenten haben kann, die ausgewählt sind aus der Gruppe bestehend aus einer Methylgruppe und Halogenatomen,
oder eine 5-Indanylgruppe).

2. Medikament für die in Anspruch 1 beschriebenen Verwendungen, wobei Z eine 1,2,4-Oxadiazol-3-yl-Gruppe ist.

3. Medikament für die in Anspruch 1 beschriebenen Verwendungen, wobei Z eine 4-Oxazolylgruppe ist.

4. Medikament für die in Anspruch 1 beschriebenen Verwendungen, wobei A eine Phenylgruppe ist, die durch wenigstens eine Methylgruppe substituiert ist und ferner 1 oder 2 Substituenten haben kann, die ausgewählt sind aus der Gruppe bestehend aus einer Methylgruppe und Halogenatomen.

5. Verwendung des N-{2-[(4-substituiertes Phenyl)amino]-2-oxoethyl}tetrahydro-2H-thiopyran-4-carboxamids, das durch die in Anspruch 1 beschriebene Formel (I) repräsentiert ist, zur Herstellung eines Medikaments zur Behandlung von Genitalherpes nach der Entwicklung von Läsionen.

## Revendications

1. Médicament à utiliser dans le traitement de l'herpès génital après le développement de lésions, lequel comprend un composé de N-{2-[(phényle substitué-4)amino]-2-oxoéthyl)tétrahydro-2H-thiopyran-4-carboxamide, représenté par la formule générale suivante (I) en tant que principe actif 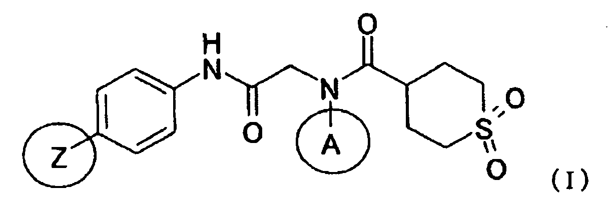 (dans la formule les symboles ont la signification suivante :
Z : est un groupe 1,2,4-oxadiazol-3-yle ou 4-oxazolyle,
A : est un groupe phényle qui est substitué par au moins un groupe méthyle et peut avoir en outre 1 ou 2 substituants sélectionnés parmi le groupe consistant en un groupe méthyle et des atomes d'halogène ou un groupe 5-indanyle).

2. Le médicament destiné aux usages décrits dans la revendication 1, où Z est un groupe 1,2,4-oxadiazol-3-yle.

3. Le médicament destiné aux usages décrits dans la revendication 1, où Z est un groupe 4-oxazolyle.

4. Le médicament destiné aux usages décrits dans la revendication 1, où A est un groupe phényle qui est substitué par au moins un groupe méthyle et peut avoir en outre 1 ou 2 substituants sélectionnés parmi le groupe consistant en un groupe méthyle et des atomes d'halogène.

5. Utilisation du N-{2-[(phényle substitué-4)amino]-2-oxoéthyl)tétrahydro-2H-thiopyran-4-carboxamide représenté par la formule (I) décrite dans la revendication 1, dans la fabrication d'un médicament pour le traitement de l'herpès génital après le développement de lésions.
